# EUROPEAN PATENT APPLICATION

(11) **EP 4 830 671 A1**
(43) Date of publication of application: **28.10.2026**
(21) Application number: 24912305.0
(22) Date of filing: 09.12.2024
(51) Int. Cl.: A61F 2/852

(54) **STENT AND METHOD FOR IMAGING STENT**

(30) Priority: 25.12.2023 JP 2023218654
(71) Applicant: T.G. Medical Inc., Tokyo 103-0023 (JP)
(72) Inventor: MIKI, Kohei, Tokyo 103-0023 (JP)
(74) Representative: Appleyard Lees IP LLP
(86) International application number: PCT/JP2024/043336
(87) International publication number: WO 2025/142416

(57) **Abstract**

Provided are a stent, and a method for imaging a stent, with which expansion in diameter can be appropriately verified. A stent 1 is used in order to expand a biological lumen, the stent comprising: a first stent body 10 in which a plurality of outer cells 12 surrounded by a plurality of first struts 11 are arranged in the circumferential direction and the center-axis direction; and a second stent body 20 in which a plurality of second cells surrounded by a plurality of second struts 21 are arranged in the circumferential direction and the central axis-direction, and in which at least a region of the second stent body 20 for expanding a biological lumen is inserted into the first stent body 10. At least part of the second stent body 20 is provided with a second stent marker part 121 having high radiopacity.

## Description

### TECHNICAL FIELD

The present disclosure relates to a stent and a method for capturing an image of a stent.

### BACKGROUND ART

When stenosis occurs in living body organs with a lumen structure, such as blood vessels, trachea, and intestines, a cylindrical mesh stent is used in order to ensure the patency of a lesion area by expanding a lumen of a stenotic part. The stent is expanded (deployed) inside the lumen structure and hence the lumen structure is expanded.

Further, an opaque member (so-called marker) which is highly opaque to radiation such as X-rays is provided in the stent in order to check a position or the like of the stent inside the lumen structure when the stent is arranged in the lumen structure (see Patent Document 1). With such a stent, it is possible to visually recognize the opaque member provided in the stent by radiation irradiation, and it is possible to improve the operability of the stent.

Further, as a stent having a larger surface area and having excellent shape followability to a vascular structure and excellent diameter-narrowing performance, a stent including a first stent body and a second stent body to be inserted into the first stent body (hereinafter, also referred to as a double-layer stent) has been disclosed (see Patent Document 2).

### Citation List

### Patent Documents

Patent Document 1: Japanese Unexamined Patent Application, Publication No. 2014-171893
Patent Document 2: PCT International Publication No. WO2022/085313

### DISCLOSURE OF THE INVENTION

### Problems to be Solved by the Invention

However, in the double-layer stent, the first stent body arranged outside and the second stent body arranged inside are independently expanded. Thus, for example, in a state in which a marker provided only on the first stent body, it can be observed under X-ray fluoroscopy that the first stent body is expanded, but it cannot be confirmed that the second stent body is adequately expanded. In an application of the stent to be deployed and temporarily placed in the narrowed blood vessel, there is a probability that incomplete expansion of the stent makes it impossible to sufficiently expand the narrowed blood vessel or impedes the blood flow.

A problem of the present disclosure is to provide a stent of which expansion can be adequately checked, and a method for capturing an image of a stent.

### Means for Solving the Problems

The present disclosure addresses the above-described problem by the following solution. Note that for the sake of easy understanding, the present disclosure will be described with reference numerals corresponding to an embodiment, but the disclosure is not limited thereto.

A first aspect of the present disclosure is directed to a stent (1, 1B, 1C, 1D, 1E, 1F) including: a first stent body (10) in which a plurality of first cells (12) surrounded by a plurality of first struts (11) are arrayed in a circumferential direction and a central axis direction; a second stent body (20) in which a plurality of second cells (22) surrounded by a plurality of second struts (21) are arrayed in the circumferential direction and the central axis direction and at least a region for expanding a living body lumen is inserted into the first stent body (10), in which at least a part of the second stent body (20) includes a second stent marker (121, 122, 123, 124, 125, 126) which is highly opaque to radiation.

According to a second aspect of the present disclosure, in the stent (1, 1B, 1C, 1D, 1E) of the first aspect, at a least part of the first stent body (10) includes a first stent marker (111, 112, 113, 114, 115) which is highly opaque to radiation, and at least a part of the first stent marker (111, 112, 113, 114, 115) is different positionally from the second stent marker (121, 122, 123, 124, 125) in an axial direction of the stent (1, 1B, 1C, 1D, 1E).

According to a third aspect of the present disclosure, in the stent (1F) of the first or second aspect, at least a part of the first stent body (10) includes a first stent marker (116) which is highly opaque to radiation, and at least a part of the first stent marker (116) is different from the second stent marker (126) in at least one of an interval and a number with which each of the first and second markers is arranged in the circumferential direction of the stent (1F).

According to a fourth aspect of the present disclosure, in the stent (1D, 1E) of the second or third aspect, at least a part of the first stent marker (114, 115) is different from the second stent marker (124, 125) in at least one of shape, size, and orientation.

A fifth aspect of the present disclosure is directed to a method for capturing an image of the stent (1, 1B, 1C, 1D, 1E, 1F) of any one of the first to fourth aspects in a living body lumen, the method including: capturing an image of the second stent markers in a diameter-narrowed state of the stent (1, 1B, 1C, 1D, 1E, 1F) at a position where the stent (1, 1B, 1C, 1D, 1E, 1F) is to be expanded in the living body lumen; and capturing an image of the second stent markers in a diameter-expanded state of the stent (1, 1B, 1C, 1D, 1E, 1F).

A sixth aspect of the present disclosure is directed to a method for expanding a living body organ with a lumen structure using a stent (1, 1B, 1C, 1D, 1E, 1F), the stent (1, 1B, 1C, 1D, 1E, 1F) including: a first stent body (10) in which a plurality of first cells (12) surrounded by a plurality of first struts (11) are arrayed in a circumferential direction and a central axis direction; a second stent body (20) in which a plurality of second cells (22) surrounded by a plurality of second struts (21) are arrayed in the circumferential direction and the central axis direction and at least a region for expanding a living body lumen is inserted into the first stent body (10), in which at least a part of the second stent body (20) includes a second stent marker (121, 122, 123, 124, 125, 126) which is highly opaque to radiation, the method including: delivering the stent (1, 1B, 1C, 1D, 1E, 1F) sheathed in a catheter (C) in a diameter-narrowed state together with the catheter (C), through the living body organ, to a position where the living body organ is to be expanded, and capturing an X-ray transmission image of the second stent markers (121, 122, 123, 124, 125, 126) in a diameter-narrowed state of the stent; retracting the catheter (C) to a proximal side, starting to expand the stent (1, 1B, 1C, 1D, 1E, 1F) from a distal side, and capturing an X-ray transmission image of the second stent markers (121, 122, 123, 124, 125, 126) while moving the stent; completing expansion of the stent (1, 1B, 1C, 1D, 1E, 1F), and capturing an X-ray transmission image of the second stent markers (121, 122, 123, 124, 125, 126) in a diameter-expanded state of the stent; and housing the expanded stent (1, 1B, 1C, 1D, 1E, 1F) in the catheter (C) again, and capturing an X-ray transmission image of the second stent markers (121, 122, 123, 124, 125, 126) in a diameter-narrowed state of the stent.

### Effects of the Invention

According to the present disclosure, there can be provided a stent of which expansion can be adequately checked, and a method for capturing an image of a stent.

### BRIEF DESCRIPTION OF THE DRAWINGS

FIG. 1 is a side view of a stent 1 of a first embodiment;
FIG. 2 is a perspective view of the stent 1 shown in FIG. 1;
FIG. 3A is a virtual development view of a part of a first stent body 10 of the first embodiment;
FIG. 3B is a virtual development view of a part of a second stent body 20 of the first embodiment;
FIG. 3C is a virtual development view of a part of the stent 1 of the first embodiment;
FIG. 4A is a diagram for describing an outer diameter D1 of the first stent body 10 alone;
FIG. 4B is a diagram for describing an outer diameter D2 of the second stent body 20 alone;
FIG. 5 is a diagram showing a process of inserting the second stent body 20 into the first stent body 10;
FIG. 6 is a sectional view in which the stent 1 is cut at a position of an arrow s1-s1 in FIG. 1;
FIG. 7 is a diagram for describing an internal state when the stent 1 is bent;
FIG. 8 is an enlarged view of a distal LD2 side end portion of the first stent body 10 in a state before a first stent marker 111 is attached thereto;
FIG. 9 is a side view of a stent 1B of a second embodiment;
FIG. 10 is a side view of a stent 1C of a third embodiment;
FIG. 11 is a side view of a stent 1D of a fourth embodiment;
FIG. 12 is a side view of a stent 1E of a fifth embodiment;
FIG. 13 is a side view of a stent 1F of a sixth embodiment;
FIG. 14A is a virtual development view of a part of a first stent body 10 of the sixth embodiment;
FIG. 14B is a virtual development view of a part of a second stent body 20 of the sixth embodiment;
FIG. 14C is a virtual development view of a part of the stent 1F of the sixth embodiment;
FIG. 15 is a diagram schematically showing an example of an image captured in capturing an image of a second stent marker 121 in a diameter-narrowed state of the stent 1 in a position where the stent 1 is to be expanded in the living body lumen;
FIG. 16 is a diagram schematically showing an example of an image captured in capturing an image of the second stent marker 121 in an intermediate state while transitioning from a diameter-narrowed state to a diameter-expanded state of the stent 1 in the position where the stent 1 is to be expanded in the living body lumen;
FIG. 17 is a diagram schematically showing an example of an image captured in capturing an image of the second stent marker 121 in a diameter-expanded state of the stent 1 in the position where the stent 1 is expanded in the living body lumen;
FIG. 18 is a diagram showing an example of an image captured in capturing an image in a diameter-narrowed state of the stent;
FIG. 19 is a diagram showing an example of an image captured in capturing an image while transitioning a state of the stent; and
FIG. 20 is a diagram showing an example of an image captured in capturing an image in a diameter-expanded state of the stent.

### PREFERRED MODE FOR CARRYING OUT THE INVENTION

Hereinafter, embodiments of a stent will be described. Note that each of the drawings attached to the present specification shows a schematic view and the shape, scale, longitudinal-lateral dimensional ratio, etc. are changed or exaggerated as compared to actual shape, scale, longitudinal-lateral dimensional ratio, etc. Moreover, in the drawings, hatching showing the cross-section of members may be omitted where appropriate.

In the present specification etc., terms specifying shapes, geometric conditions, and the degrees thereof, such as "perpendicular" and "direction", include not only exact meanings of these terms, but also ranges taken as being substantially perpendicular and being substantially in a direction. In description of the present specification etc., a (proximal) side close to a practitioner will be referred to as a side LD1 (or a proximal LD1 side), a (distal) side distant from the practitioner will be referred to as a side LD2 (or a distal LD2 side) in an axial direction (a longitudinal direction) LD. And a direction perpendicular to the axial direction LD will be referred to as a radial direction RD. Moreover, in description of the present specification etc., a direction in which cells are arrayed will be referred to as a circumferential direction OD. The circumferential direction may be not only the radial direction RD, but also a direction inclined with respect to the radial direction RD.

### (First Embodiment)

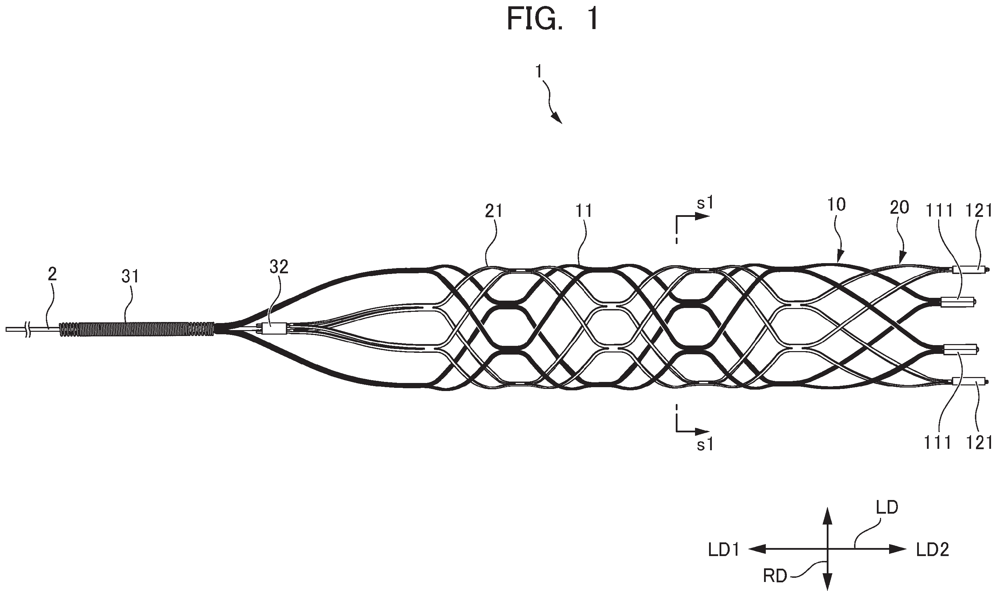

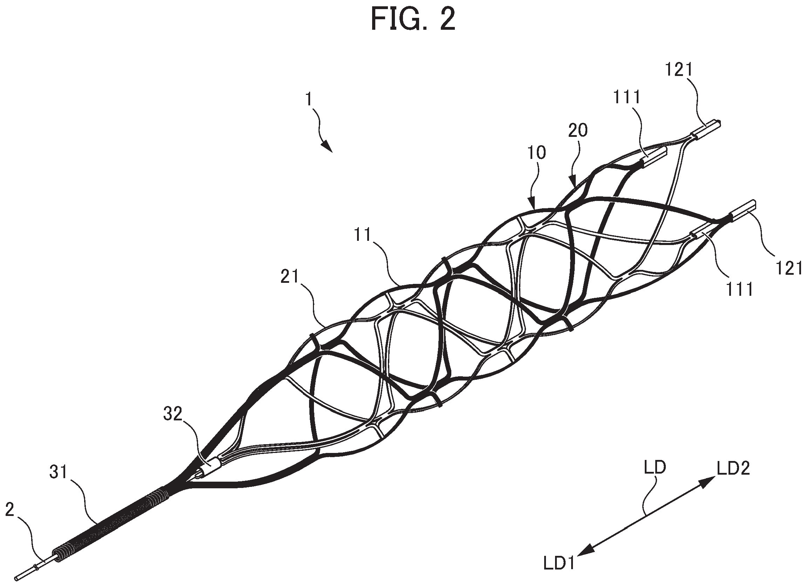

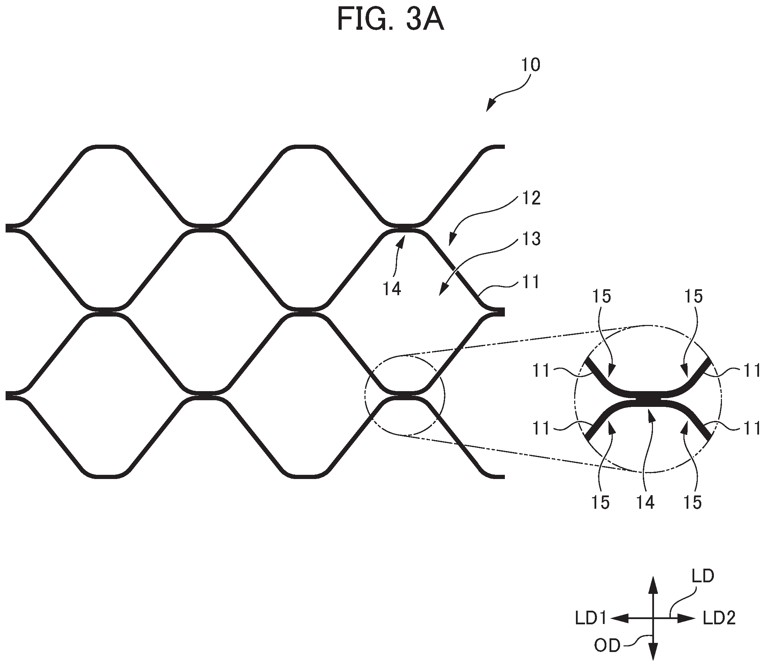

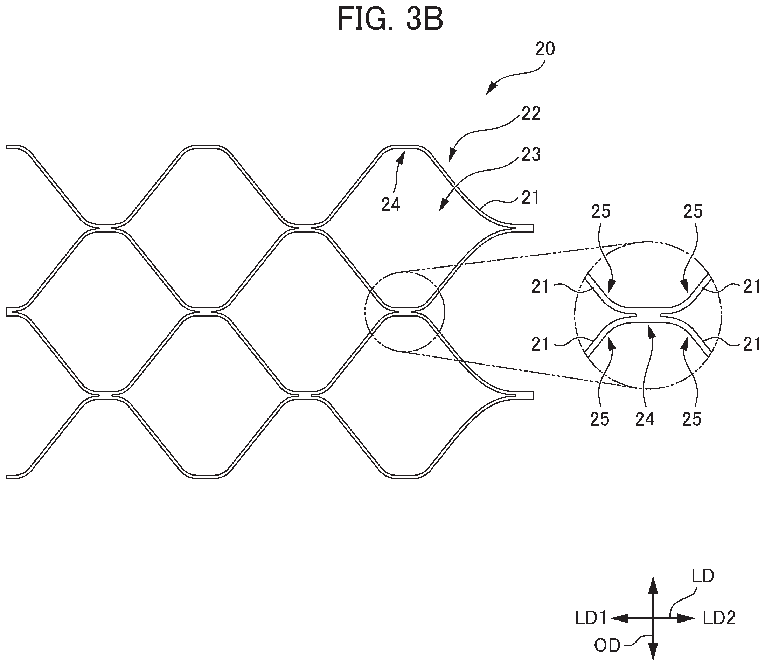

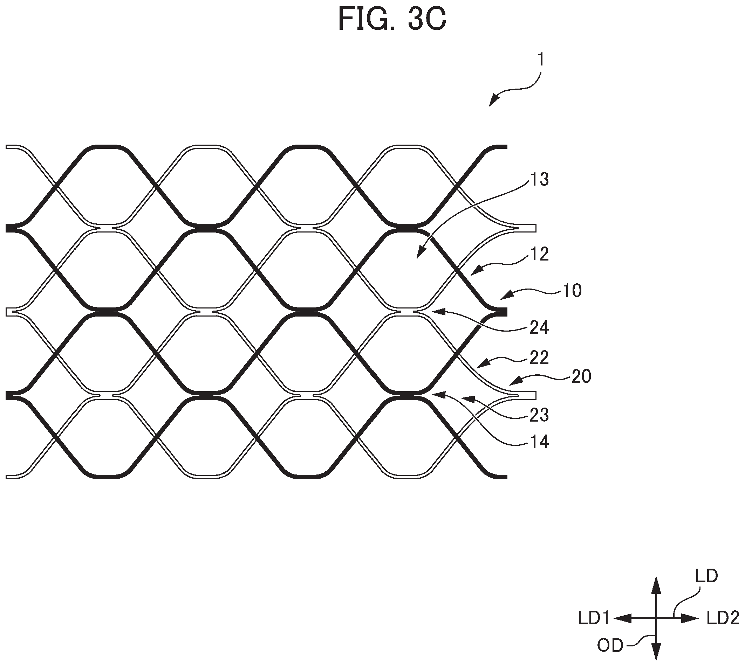

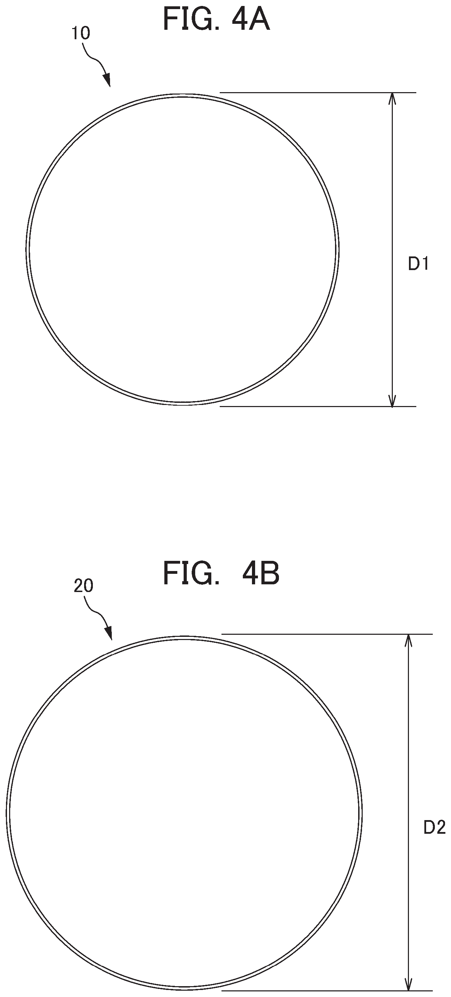

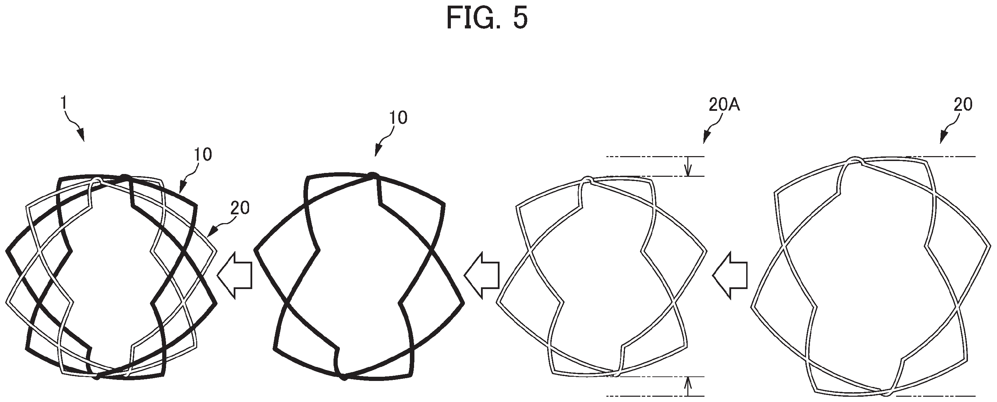

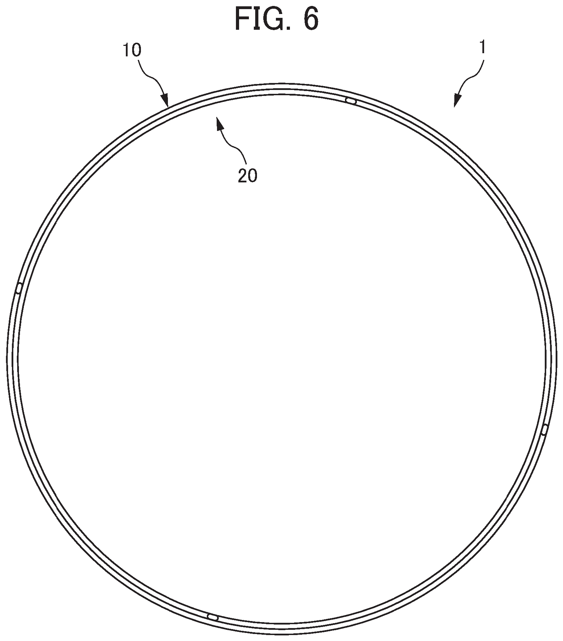

FIG. 1 is a side view of a stent 1 of a first embodiment. FIG. 2 is a perspective view of the stent 1 shown in FIG. 1. FIG. 3A is a virtual development view of a part of a first stent body 10 of the first embodiment. FIG. 3B is a virtual development view of a part of a second stent body 20 of the first embodiment. FIG. 3C is a virtual development view of a part of the stent 1 of the first embodiment. FIG. 4A is a diagram for describing an outer diameter D1 of the first stent body 10 alone. FIG. 4B is a diagram for describing an outer diameter D2 of the second stent body 20 alone. FIG. 5 is a diagram showing a process of inserting the second stent body 20 into the first stent body 10. FIG. 6 is a sectional view in which the stent 1 is cut at a position of an arrow s1-s1 in FIG. 1.

In the drawings, for the sake of easy distinguishing of the first stent body 10 and the second stent body 20, a strut of the first stent body 10 is indicated by black, and a strut of the second stent body 20 is indicated by white. In the present specification etc., the "cell" indicates a portion surrounded by struts forming a stent.

"Cells" may be identical to each other or different from each other in shape and size. The "strut" indicates a portion made of wire-like material. In the present specification etc., a cell opening will be referred to as an "opening portion", and a portion where struts of adjacent cells are connected to or overlap with each other will also be referred to as an "overlapping portion".

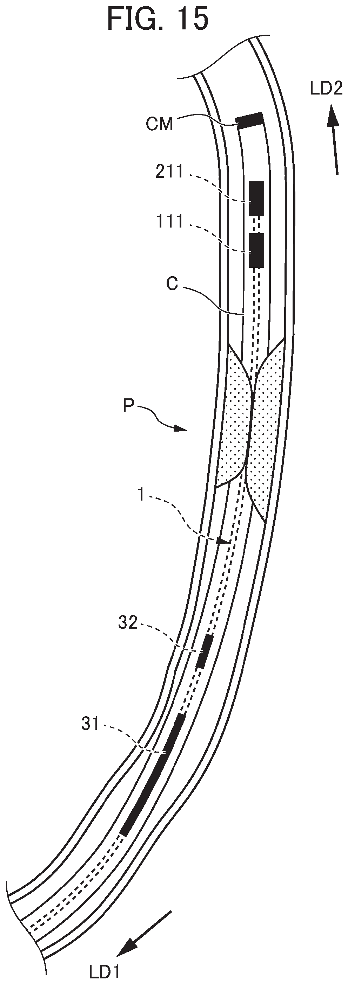

The stent 1 of the first embodiment may be used, for example, as follows: the stent 1 is sheathed (housed) in a catheter C (see FIGS. 15 to 17), pushed out of the catheter and expanded in a vascular lumen, such that the stent 1 expands a narrowed or occluded blood vessel. The stent 1 is used for an application to be retrieved from the body after having been temporarily placed in a blood vessel. Alternatively, the stent 1 may be used while remained in the blood vessel without being retrieved from the body. When the stent 1 is used for such an application, it may be preferable that a push wire 2 and the stent 1 are configured to be separable from each other through the operation by a practitioner.

As shown in FIGS. 1 and 2, the stent 1 is in a substantially cylindrical shape in a diameter-expanded state. The stent 1 is in an elongated cylindrical shape in a diameter-narrowed state. The push wire 2 is connected to a proximal LD1 side end portion of the stent 1. A method for connecting the proximal side end portion of the stent 1 and the push wire 2 to each other is not particularly limited as long as the connection method is used for general medical equipment. Examples of the method may include welding, UV bonding, and silver solder infiltration.

The practitioner pushes or pulls the push wire 2 via an operation unit (not shown) outside the body that is coupled to the proximal LD1 side of the push wire 2, thereby moving the stent 1 back and forth in the catheter or the blood vessel. As shown in FIGS. 15 to 20 described later, the catheter C is pulled (moved back) and pushed (moved forth) without moving the push wire 2 (the stent 1), making it possible to remove the stent 1 from the catheter C to expand the stent 1 and resheathe the stent in the catheter C.

The stent 1 includes the first stent body 10 and the second stent body 20, each of which is a substantially cylindrical structure. The stent 1 is a stent having such a double-layer structure in which the second stent body 20 is inserted into the first stent body 10 in at least a region that expands a narrowed site of a living body lumen. Here, the region that expands a narrowed site of a living body lumen refers to a region substantially contributing to expansion of the narrowed site during use. This region corresponds to a region expanding in a substantially cylindrical shape in a diameter-expanded state shown in FIGS. 1 and 2 in the stent 1 of the present embodiment. Moreover, the above-described "at least" means that in the above-described region that expands a narrowed site, the second stent body 20 is required to be inserted into the first stent body 10. Note that the region in which the second stent body 20 is inserted into the first stent body 10 is not limited to the above-described region that expands a narrowed site (in the example of the present embodiment, a region expanding in a substantially cylindrical shape). In the present embodiment, the second stent body 20 is inserted into the first stent body 10 immediately before the position where the first stent body 10 and the second stent body 20 are coupled to the push wire 2.

The first stent body 10 and the second stent body 20 are not coupled to each other in the radial direction. Specifically, the first stent body 10 and the second stent body 20 are indirectly coupled to each other via the push wire 2, but are not coupled to each other in the other region. Thus, the first stent body 10 and the second stent body 20 are independently and flexibly deformable. Also in a diameter-expanded state, the first stent body 10 and the second stent body 20 just closely contact to each other in the radial direction but are not coupled (connected) to each other in the radial direction. Thus, the first stent body 10 and the second stent body 20 are independently deformable, but do not restrict deformation of each other.

The proximal LD1 side of the first stent body 10, the proximal LD1 side of the second stent body 20, and the distal LD2 side of the push wire 2 are directly connected to each other. Here, "directly connected" refers to, for example, a form in which the proximal LD1 side of the first stent body 10 and the distal LD2 side of the push wire 2 are connected to each other and the proximal LD1 side of the second stent body 20 and the distal LD2 side of the push wire 2 are connected to each other. In this case, a position where the proximal LD1 side of the first stent body 10 is connected to the push wire 2 may be the same as or different from a position where the proximal LD1 side of the second stent body 20 is connected to the push wire 2.

Alternatively, the proximal LD1 side of the first stent body 10, the proximal LD1 side of the second stent body 20, and the distal LD2 side of the push wire 2 may be indirectly connected to each other. Here, "indirectly connected" refers to, for example, a form in which the proximal LD1 side of the first stent body 10 and the distal LD2 side of the push wire 2 are connected to each other and the proximal LD1 side of the first stent body 10 and the proximal LD1 side of the second stent body 20 are connected to each other (or a connection form in which the two stent bodies are connected in an inverse relationship). In this case, the proximal LD1 side of the second stent body 20 is indirectly connected to the push wire 2 via the proximal LD1 side of the first stent body 10. Alternatively, there may be another form in which the proximal LD1 side of the first stent body 10 and the proximal LD1 side of the second stent body 20 are connected to each other via a pipe-like sleeve (not shown) inserted over the distal LD2 side of the push wire 2. In this case, the proximal LD1 side of the first stent body 10 and the proximal LD1 side of the second stent body 20 are indirectly connected to the distal LD2 side of the push wire 2 via the sleeve. As described above, in the present specification, "indirectly connected" includes a form in which one of the proximal LD1 side of the first stent body 10 and the proximal LD1 side of the second stent body 20 is directly connected to the push wire 2, and the other stent body, which is not connected directly to the push wire 2, is indirectly connected to the push wire 2 via the stent body directly connected to the push wire 2. Examples of a method for connecting the proximal side end portion of each stent body and the push wire 2 to each other may include welding, UV bonding, and silver solder infiltration, but is not particularly limited as long as the connection method is used for general medical equipment.

As described later, the stent 1 of the first embodiment is produced in such a manner that the second stent body 20, which has a greater outer diameter than that of the first stent body 10 in a natural state, is inserted into the first stent body 10 in a diameter-narrowed state. Thus, the second stent body 20 constantly presses the first stent body 10 outward in the radial direction RD, so that the first stent body 10 and the second stent body 20 is closely in contact with each other.

As shown in FIG. 1, the proximal LD1 side end portions of the first stent body 10 and the second stent body 20, which are tapered and narrowed, are connected to the push wire 2. Thus, the second stent body 20 is restricted from moving in the axial direction relative to the first stent body 10.

In the first stent body 10, as shown in FIG. 3A, a plurality of outer cells (first cells) 12 surrounded by first struts 11 are arrayed in a circumferential direction OD. In the first stent body 10, the plurality of outer cells 12 are arrayed in the axial direction LD. That is, the first stent body 10 has such a pattern that the plurality of outer cells 12 are arrayed in the circumferential direction OD and the axial direction LD. Moreover, the outer cells 12 adjacent to each other in the circumferential direction OD are coupled to each other at an overlapping portion 14.

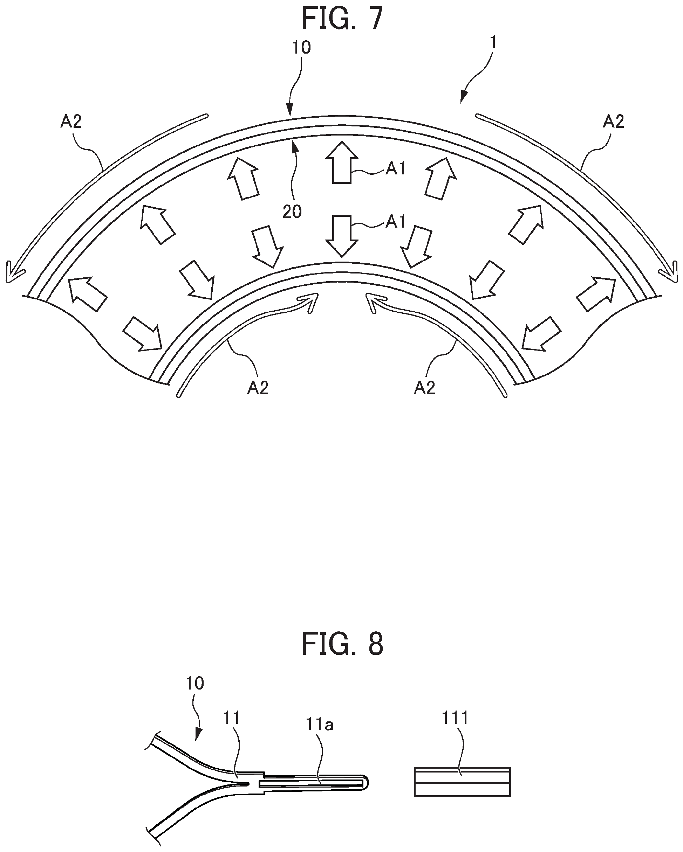

The overlapping portion 14 is a portion at which the first struts 11 for the four outer cells 12 adjacent to each other are connected. The overlapping portion 14 has a substantially rectangular shape elongated in the axial direction LD. The first struts 11 are individually connected to four corners of the overlapping portion 14. In each of the first struts 11, a curved portion 15 is formed at a portion connected to the overlapping portion 14. Thus, when the expanded stent 1 is bent into a substantially U shape (see FIG. 7), each of the first struts 11 connected to the overlapping portion 14 is independently deformable. Therefore, the outer cells 12 arrayed in the circumferential direction OD bend more flexibly. As described above, the outer cells 12 arrayed in the circumferential direction OD flexibly bend, and therefore, the first stent body 10 is excellent in shape followability and diameter-narrowing performance.

In the second stent body 20, as shown in FIG. 3B, a plurality of inner cells (second cells) 22 surrounded by second struts 21 are arrayed in the radial direction (the circumferential direction) RD. The plurality of inner cells 22 are arrayed in the axial direction LD. That is, the second stent body 20 has such a pattern that the plurality of inner cells 22 are arrayed in the circumferential direction OD and the axial direction LD. The inner cells 22 adjacent to each other in the circumferential direction OD are coupled to each other at an overlapping portion 24.

The overlapping portion 24 is a portion at which the second struts 21 for the four inner cells 22 adjacent to each other are connected. The overlapping portion 24 has a substantially rectangular shape elongated in the axial direction LD. The second struts 21 are individually connected to four corners of the overlapping portion 24. In each of the second struts 21, a curved portion 25 is formed at a portion connected to the overlapping portion 24. Thus, when the expanded stent 1 is bent into a substantially U shape, each of the second struts 21 connected to the overlapping portion 24 is independently deformable. Therefore, the inner cells 22 arrayed in the circumferential direction OD bend more flexibly. As described above, the inner cells 22 arrayed in the circumferential direction OD bend flexibly, and therefore, the second stent body 20 is excellent in shape followability and diameter-narrowing performance.

As shown in FIGS. 3A and 3B, in the stent 1 of the first embodiment, an outer cell 12 and an inner cell 22 have the same size, shape, and arrangement except for the distal LD2 side, as one example. That is, in the first embodiment, the patterns shown in FIG. 3A and FIG. 3B are substantially the same except for the distal LD2 side.

As shown in FIG. 3C, the first stent body 10 and the second stent body 20 are arranged to overlap with each other in a natural state such that the overlapping portion 24 between the adjacent inner cells 22 is arranged in an opening portion 13 of the outer cell 12. Specifically, one overlapping portion 24 between the adjacent inner cells 22 overlaps with one opening portion 13 of the outer cell 12. Thus, the density of the struts can be increased over the entire stent, and the surface area of the stent 1 can be increased. Moreover, the overlapping portion 14 between the adjacent outer cells 12 has a configuration as shown in FIG. 3A, and the overlapping portion 24 between the adjacent inner cells 22 has a configuration as shown in FIG. 3B, and therefore, the stent 1 of the present embodiment is excellent in shape followability and diameter-narrowing performance. Moreover, the stent 1, which is configured such that the outer cells 12 and the inner cells 22 have the configuration described above, produces effects that the narrowed stent 1 is easily sheathed in a catheter and the stent 1 expanded in a blood vessel is easily resheathed in a catheter.

In the first embodiment, a relationship between an outer diameter D1 of the first stent body 10 before assembly and an outer diameter D2 of the second stent body 20 before assembly is set to D1 < D2. Thus, as shown in FIG. 5, a second stent body 20A obtained by narrowing the diameter of the second stent body 20 is inserted into the first stent body 10. As shown in FIG. 6, the second stent body 20 closely contacts the inside of the first stent body 10 in the circumferential direction OD due to expansive force of the second stent body 20 itself.

Thus, in the axial direction LD (see FIG. 1) of the stent 1, it is less likely that the positions of the first stent body 10 and the second stent body 20 are misaligned relative to each other. Note that for the sake of easy understanding, FIG. 5 shows only an annular circumferential cell line of each stent body.

In the first embodiment, the second stent body 20 inserted into the first stent body 10 in the diameter-narrowed state constantly presses the first stent body 10 outward in the radial direction RD. Consequently, even if the first stent body 10 and the second stent body 20 are not coupled to each other in the radial direction, the first stent body 10 and the second stent body 20 can closely contact each other. Moreover, since the first stent body 10 and the second stent body 20 are not coupled to each other in the radial direction in the stent 1, the first stent body 10 and the second stent body 20 are independently and flexibly deformable. Further, the stent 1 has the total expansive force created by the first stent body 10 and the second stent body 20. Thus, the stent 1 can have a greater ratio of the expansive force to the surface area than that of a stent having a single-layer structure.

The stent 1 (the first stent body 10, the second stent body 20) may be made of a material having a high biological compatibility. The stent 1 is preferably made of, but is not particularly limited to, a material having superelastic properties, such as nickel titanium (Ni-Ti) alloy. The first stent body 10 and the second stent body 20 may be produced, for example, in such a manner that substantially cylindrical tubes made of the above-described material are machined with laser.

The stent 1 may be loaded with a medical agent such as a physiologically active substance (a medical agent for inhibiting intima thickening, etc.), and specifically, may carry the medical agent so as to dissolve out the medical agent in the lumen structure.

For example, the first stent body 10 and the second stent body 20 are produced by machining tubes having outer diameters of about 0.4 to 3 mm with laser to form mesh patterns, and then, stretching the mesh patterns in the radial direction to expand to desired diameters. As described above, the second stent body 20 is inserted into the first stent body 10, and subsequently, the stent 1 having the double-layer structure can be produced. The stent 1 having the double-layer structure is sheathed in the catheter (not shown) while being narrowed in the radial direction from the natural state. The stent 1, which is made of, e.g., an elastic material such as superelastic alloy or shape-memory alloy, expands in diameter due to the self-expanding force when the stent 1 sheathed in the catheter is pushed out.

Next, an effect in a case where the stent 1 of the first embodiment is removed after having been temporarily placed in a bent blood vessel will be described herein.

As described above, the stent 1 of the present embodiment is a stent having such a double-layer structure in which the second stent body 20 is inserted into the first stent body 10. The stent 1 is configured such that the first stent body 10 and the second stent body 20 are independently deformable on the respective layers, and therefore, the above-described peculiar excellent effects can be exhibited. On the other hand, the stent 1 is configured such that the first stent body 10 and the second stent body 20 are independently deformable on the respective layers, and therefore, it may occur that only one stent body does not expand adequately. Consequently, in the stent having a double-layer structure, the importance of checking the diameter-expanded state of the stent with the marker is higher than that of the conventional stent having a one-layer structure.

The stent 1 of the first embodiment includes, as markers, a first connection portion marker 31, a second connection portion marker 32, a first stent marker 111, and a second stent marker 121. These markers each are a member serving as a mark for checking the position and the state of the stent 1 under observation by radiation in the tubular organ such as a blood vessel, and are formed of a material which is highly opaque to radiation (has a high radiopacity). Here, the radiation opacity indicates that, under observation using radiation during operation, the markers are more highly opaque to radiation than portions other than the markers in the stent 1 (other portions having no radiation opacity), and are opaque to radiation to the extent that the markers can be observed as images. The "radiation opacity" is required to obtain the "visibility" and "radiopacity" under observation using radiation.

First, descriptions will be given of configurations of conventional markers that can be used as: the first connection portion marker 31, the second connection portion marker 32, the first stent marker 111, and the second stent marker 121 of the present embodiment (hereinafter, simply referred to as markers); and various markers which will be described in the second embodiment and thereafter.

As a highly radiopaque material, which can be used as a marker, a metal or a synthetic resin may be used, and examples of such a material may include gold, tantalum, platinum, tungsten, iridium, and platinum tungsten, and an alloy containing these materials. Moreover, examples of such a material may include a polymer material having radiation opacity to which a radiopaque filler or the like is added. When the marker is formed of a linear member, a wire made of a composite member having a coaxial core material made of the above-described metal material in a wire made of nickel titanium can be used, for example.

As a method of attaching the markers to the stent 1, a conventional known manufacturing method used when the markers are attached to the stent, such as gold-tin solder, silver-tin solder, laser welding, mechanical pressing, bonding with resin, can be used where appropriate.

Examples of specific forms that can be used as the markers of the present embodiment include a "crimping marker", a "coil marker", a "radiopaque wire marker", and a "radiopaque strut". Note that the above-described exemplary forms and the designations are exemplified by being classified and called for purposes of convenience by the applicant, and various conventional forms of markers can be used.

The crimping marker is, for example, a form in which a highly radiopaque marker component having a ring shape or a substantially cylindrical shape is fixed to a strut or the like by crimping.

The coil marker, which is formed of a linear member of a highly radiopaque material into a coil shape, is wound around a strut to be fixed by soldering, bonding, or the like.

The radiopaque wire marker , in which a highly radiopaque wire is used as a marker, is provided along or wound around a strut or the like, and an end portion of the wire is fixed to the strut by soldering, bonding, or the like. The radiopaque wire marker is similar to the above-described coil marker, but is classified separately from the coil marker, since the wire is not wound so densely to be called a coil.

The radiopaque strut includes forms in which the strut itself is formed of a highly radiopaque material and a highly radiopaque material is tightly attached to the entire surface or a partial surface of the strut by plating, coating, or the like.

The first connection portion marker 31 is a coil marker obtained by forming a linear member of a highly radiopaque material into a coil shape, and is fixed to a joining portion of the proximal LD1 side end portion of the first stent body 10 by welding. The first connection portion marker 31 is formed in a closely-wound coil shape around the center, and is formed in an open-wound coil shape around both ends. This configuration prevents bending of the push wire 2.

The second connection portion marker 32 is a crimping marker in which a marker component made of a highly radiopaque material and formed in a circular tube is fixed to a strut by crimping, and is fixed to a coupling portion at the proximal LD1 side end portion of the second stent body 20. The second connection portion marker 32 is arranged closer on the distal LD2 side than the first connection portion marker 31.

The first connection portion marker 31 and the second connection portion marker 32 are positioned apart from each other at a predetermined distance in the axial direction LD. Such an arrangement makes it possible to correctly check the position of the proximal side LD1 end portion of each of the first stent body 10 and the second stent body 20. Moreover, the arrangement can serve as a mark of a position where the catheter C (see FIG. 15) is pulled in a diameter-expanded state as described later.

The first stent marker 111 is a crimping marker which is formed in a substantially square pipe shape, and is crimp-fixed to the distal LD2 side end portion of the first stent body 10. In the present embodiment, the first stent body 10 has two distal LD2 side end portions, and thus, two first stent markers 111 are provided thereto.

FIG. 8 is an enlarged view of a distal LD2 side end portion of the first stent body 10 in a state before a first stent marker 111 is attached thereto. A slit 11a is opened in the distal LD2 side end portion of the first stent body 10. A part of the first stent marker 111 goes into the slit 11a and engages therewith by crimping, so that the first stent marker 111 can be prevented from coming off.

The second stent marker 121 is a crimping marker which is formed in a substantially square pipe shape, and is crimp-fixed to the distal LD2 side end portion of the second stent body 20 in a manner similar to the first stent marker 111. In the present embodiment, the second stent body 20 has two distal LD2 side end portions, and thus, two second stent markers 121 are provided thereto.

In the observation by X-rays, it is difficult to visually distinguish and identify the first stent marker 111 and the second stent marker 121 only based on the shape of a marker. In the present embodiment, the first stent marker 111 is arranged to be different from the second stent marker 121 in a position in the axial direction LD of the stent. More specifically, the second stent marker 121 is arranged closer on the distal LD2 side than the first stent marker 111. In the present embodiment, the inner cells 22 on the most distal LD2 side have a shape elongated on the distal LD2 side with respect to the other cells (the inner cells 22 and the outer cells 12 on the more proximal LD1 side). Accordingly, the second stent marker 121 is arranged closer on the distal LD2 side than the first stent marker 111.

Such a configuration makes it possible to determine visually to which of a first stent marker 111 and a second stent marker 121an observed stent marker corresponds in the observation by X-rays when the first stent markers 111 and the second stent markers 121 are simultaneously observed. In other words, it is possible to visually distinguish and observe the first and second stent markers 111 and 121. Therefore, it is possible to check correctly the position of the distal LD2 side end portion of each of the first stent body 10 and the second stent body 20, and the adequately diameter-expanded state or the adequately diameter-narrowed state of each of the first stent body 10 and the second stent body 20.

Note that, in the present embodiment, the first stent markers 111 and the second stent markers 121 have the same shape and size. The second stent marker 121 is arranged closer on the distal LD2 side than the first stent marker 111 in the axial direction LD of the stent 1. That is, the first stent markers 111 and the second stent markers 121 as a whole are different from each other in a position in the axial direction of the stent 1. The present disclosure is not limited thereto, and for example, the first stent markers 111 and the second stent markers 121 may be different partly from each other in a position in the axial direction of the stent 1. More specifically, for example, a length of the second stent marker 121 in the axial direction is configured to be longer than a length of the first stent marker 111 in the axial direction. This configuration may allow to set either one or both of the proximal LD1 side end portion and the distal LD2 side end portion of each of the first stent marker 111 and the second stent marker 121 to be different from each other. Moreover, all of the first stent markers 111 and the second stent markers 121 need not be in the forms exemplified in the present embodiment, and for example, at least some of the first stent markers 111 may be arranged at the same positions as the second stent markers 121 in the axial direction LD. Similarly, some of the second stent markers 121 may be arranged at the same positions as the first stent markers 111 in the axial direction LD.

The stent 1 of the first embodiment is deployed in a lesion area of a vascular lumen, and in this manner, the vascular lumen can be expanded, the patency of the lesion area can be ensured, and blood flow can be restored. The stent 1 is removed (retrieved) from the vascular lumen in a predetermined period of time so that occurrence of restenosis or reocclusion or occurrence of a complication such as a thrombosis using an indwelling stent can be reduced. When the stent 1 is expanded or narrowed, the first stent marker 111 and the second stent marker 121 can be distinguished and recognized, and therefore, it is possible to adequately grasp the states of the first stent body 10 and the second stent body 20 and it is possible to more safely and reliably perform the operation. Since the stent 1 of the first embodiment has excellent shape followability, the high vascular preservation is obtained. Note that the stent 1 of the first embodiment can be used not only for treatment of a blood vessel but also for treatment of lumen structures such as the esophagus and large intestine.

The stent 1 of the first embodiment can also be used for treatment of a cerebrovascular spasm. Note that stents of other embodiments to be described later also produce advantageous effects similar to those of the stent 1 of the first embodiment.

### (Second Embodiment)

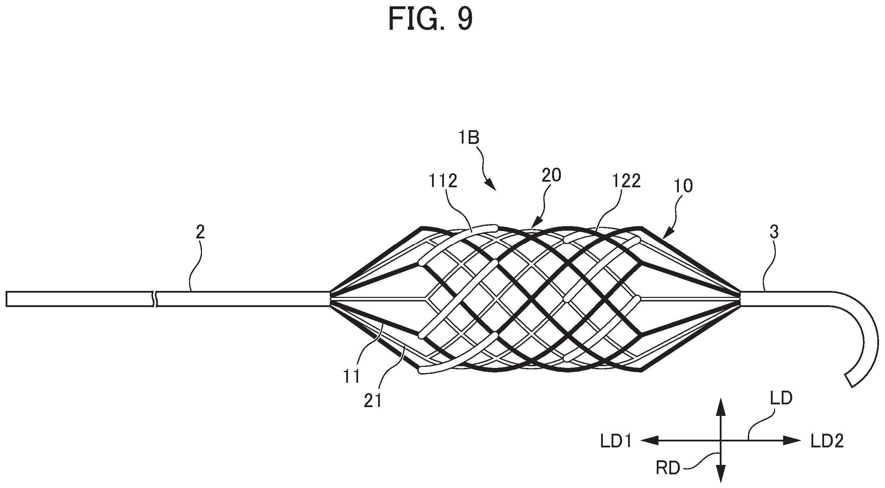

FIG. 9 is a schematic side view of a stent 1B of a second embodiment. In the stent 1B of the second embodiment, a first stent marker 112 and a second stent marker 122 are different from the first stent marker 111 and the second stent marker 121 of the first embodiment. The stent 1B of the second embodiment is similar to the stent of the first embodiment except for the number of arrays of cells and the form of the distal LD2 side. Thus, the same reference numerals are used as elements having functions similar to those of the above-described first embodiment, and overlapping description thereof will be omitted as necessary. Note that FIG. 9 shows a more simplified schematic diagram than FIG. 1 of the first embodiment. Moreover, the first connection portion marker 31 and the second connection portion marker 32 shown in the first embodiment are not shown, but they are configured similarly to the first embodiment (the same applies to other embodiments described below).

A distal end shaft 3 is provided on the distal LD2 side of the stent 1B of the second embodiment. The distal end shaft 3 has a function as a marker serving as a mark for checking the position on the distal LD2 side of the stent 1B in an X-ray transmission image, and for example, the entirety or a part of the distal end shaft 3 is formed of a material which is highly opaque to radiation. Note that the distal end shaft 3 may be made, for example, of the same material as that of a push wire 2. On the distal LD2 side of the stent 1B, the end portions of the first stent body 10 and the second stent body 20 are gradually narrowed toward a side of the distal end shaft 3, and are connected to the distal end shaft 3.

In the stent 1B of the second embodiment, the meshes are arrayed more densely than those of the first embodiment. Specifically, in the stent 1 of the first embodiment, the two outer cells 12 of the first stent body 10 and the two inner cells 22 of the second stent body 20 are arrayed in the circumferential direction. On the contrary, in the second embodiment, six outer cells 12 of a first stent body 10 and six inner cells 22 of a second stent body 20 are arrayed in the circumferential direction. Therefore, it is possible to increase a surface area of a portion contacting an inner wall of the blood vessel or the like.

The first stent marker 112 is a member serving as a mark for checking the position and the state of the first stent body 10 under observation by radiation, and is formed of a material which is highly opaque to radiation. The first stent marker 112 is provided at a position closer on the proximal LD1 side than the center with respect to a first strut 11 of the first stent body 10. For the first stent marker 112, for example, a crimping marker can be used, but the first stent marker 112 may be formed of a radiopaque wire marker or radiopaque strut.

The second stent marker 122 is a member serving as a mark for checking the position and the state of the second stent body 20 under observation by radiation, and is formed of a material which is highly opaque to radiation. The second stent marker 122 is provided at a position closer on the distal LD2 side than the center with respect to a second strut 21 of the second stent body 20. The second stent marker 122 is positioned apart from the first stent marker 112 in the axial direction LD. For the second stent marker 122, for example, a crimping marker can be used, but the second stent marker 122 may be formed of the coil marker, the radiopaque wire marker or the radiopaque strut.

In the second embodiment, the first stent marker 112 and the second stent marker 122 are arranged at different positions in the axial direction LD, and therefore, the first stent marker 112 and the second stent marker 122 can be visually distinguished and observed under observation by radiation. Therefore, it is possible to correctly check the position and the diameter-expanded state or the diameter-narrowed state for each of the first stent body 10 and the second stent body 20. Note that all of the first stent markers 112 and the second stent markers 122 need not be in the forms exemplified in the present embodiment, and for example, at least some of the first stent markers 112 may be arranged at the same positions as the second stent markers 122 in the axial direction LD. Similarly, some of the second stent markers 122 may be arranged at the same positions as the first stent markers 112 in the axial direction LD.

### (Third Embodiment)

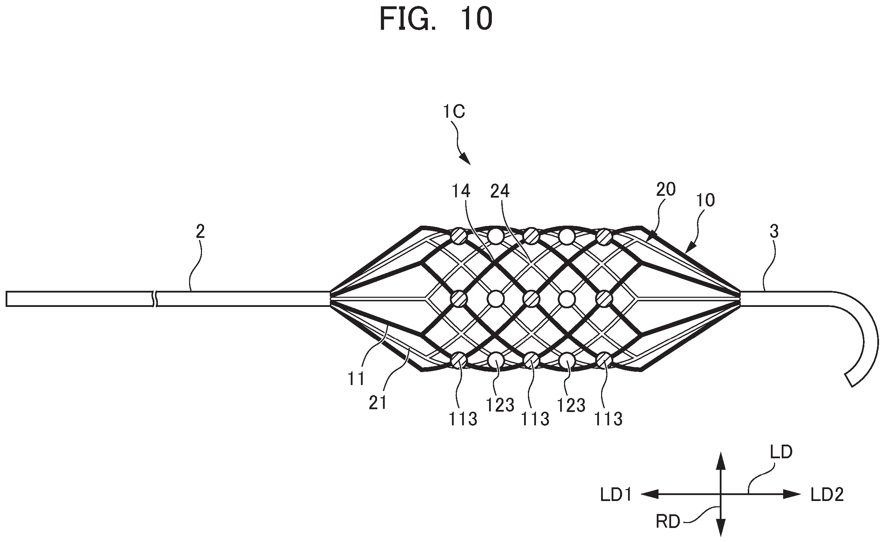

FIG. 10 is a schematic side view of a stent 1C of a third embodiment. The stent 1C of the third embodiment is similar to the stent of the second embodiment except that a first stent marker 113 and a second stent marker 123 are different from the first stent marker 112 and the second stent marker 122 of the second embodiment. Therefore, the same reference numerals are used as elements having functions similar to those of the above-described second embodiment, and overlapping description thereof will be omitted as necessary. Note that FIG. 10 shows a more simplified schematic diagram than FIG. 1 of the first embodiment.

The first stent marker part 113 is a member serving as a mark for checking the position and the state of a first stent body 10 under observation by radiation, and is formed of a material which is highly opaque to radiation. The first stent marker 113 is arranged on an overlapping portion 14 of the first stent body 10. First stent markers 113 are arranged on all overlapping portions 14 that are adjacent to each other in the circumferential direction OD. Moreover, the first stent markers 113 are not arranged on all of the overlapping portions 14 in the axial direction LD, and are arranged according to a predetermined rule.

The second stent marker 123 is a member serving as a mark for checking the position and the state of a second stent body 20 under observation by radiation, and is formed of a material which is highly opaque to radiation. The second stent marker 123 is arranged on an overlapping portion 24 of the second stent body 20. Second stent markers 123 are arranged on all overlapping portions 24 that are adjacent to each other in the circumferential direction OD. Moreover, the second stent markers 123 are not arranged on all of the overlapping portions 24 in the axial direction LD, and are arranged according to a predetermined rule.

The first stent markers 113 and the second stent markers 123 are different in: the first stent markers 113 are attached to the first stent body 10; and the second stent markers 123 are attached to the second stent body 20. However, it is difficult to distinguish markers alone from each other since the markers themselves have the similar form. In the present embodiment, the first stent markers 113 and the second stent markers 123 are arranged at different positions in the axial direction of the stent 1C according to a predetermined rule, so that each of the markers can be visually distinguished and observed under observation by radiation.

Specifically, in the present embodiment, the first stent markers 113 are arranged on most proximal LD1 side and most distal LD2 side in the axial direction LD of the stent 1C, and are further arranged in substantially the center in the axial direction LD. On the other hand, the second stent markers 123 are arranged at positions in the axial direction LD where the first stent markers 113 arranged in three rows in the axial direction LD are not arranged and at positions (rows) sandwiched between the first stent markers 113. That is, as shown in FIG. 10, the first stent markers 113 and the second stent markers 123 are alternately arranged in the axial direction LD. Therefore, if this arrangement rule is known, it is possible to visually distinguish and observe the first stent markers 113 and the second stent markers 123 under observation by radiation. Note that all of the first stent markers 113 and the second stent markers 123 need not be in the forms exemplified in the present embodiment, and for example, at least some of the first stent markers 113 may be arranged at the same positions (the same rows) as the second stent markers 123 in the axial direction LD. Similarly, some of the second stent markers 123 may be arranged at the same positions (in the same rows) as the first stent markers 113 in the axial direction LD.

### (Fourth Embodiment)

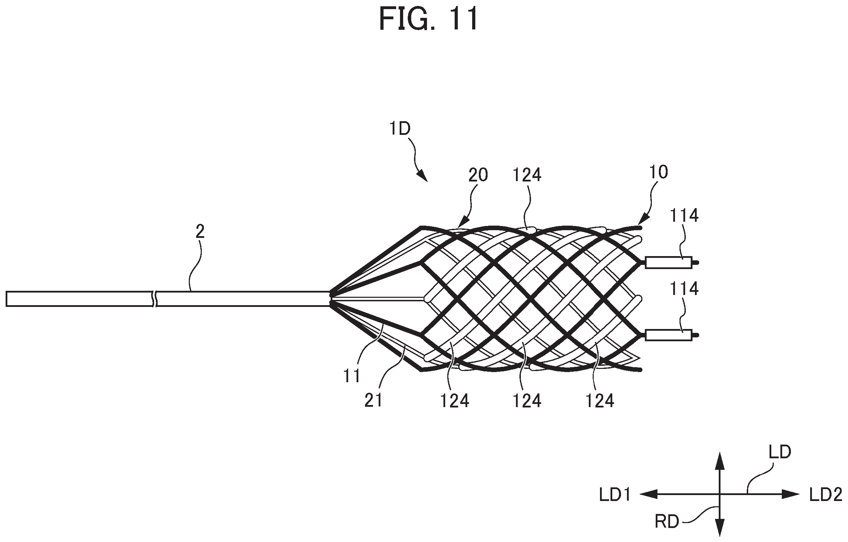

FIG. 11 is a schematic side view of a stent 1D of a fourth embodiment. In the stent 1D of the fourth embodiment, the distal LD2 side has a shape of an open end as in the first embodiment. In the stent 1D of the fourth embodiment, a first stent marker 114 and a second stent marker 124 are different from the first stent marker 112 and the second stent marker 122 of the second embodiment. The stent 1D of the fourth embodiment is similar to the stent of the second embodiment except for the above-described different point. Therefore, the same reference numerals are used as elements having functions similar to those of the above-described second embodiment, and overlapping description thereof will be omitted as necessary. Note that FIG. 11 shows a more simplified schematic diagram than FIG. 1 of the first embodiment.

The first stent marker 114 is a member serving as a mark for checking the position and the state of a first stent body 10 under observation by radiation, and is formed of a material which is highly opaque to radiation. The first stent marker 114 is a marker substantially similar to the first stent marker 111 of the first embodiment, and is a crimping marker that is crimp-fixed to the distal LD2 side end portion of the first stent body 10. FIG. 11 shows only two first stent markers 114, but the number of the first stent markers can be changed where appropriate.

The second stent marker 124 is a member serving as a mark for checking the position and the state of a second stent body 20 under observation by radiation, and is formed of a material which is highly opaque to radiation. Second stent markers 124 are arranged along only one direction in second struts 21 crossing and extending in two directions in the second stent body 20. For the second stent marker 124, for example, a crimping marker can be used, but the second stent marker 124 may be formed of the coil marker, the radiopaque wire marker or the radiopaque strut.

In the fourth embodiment, the first stent markers 114 and the second stent markers 124 are arranged at different positions in the axial direction LD, and further, the forms (shape, size, and orientation) to be observed are also greatly different from each other. This makes it possible to visually distinguish and observe the first stent marker 114 and the second stent marker 124 more reliably under observation by radiation. Note that all of the first stent markers 114 and the second stent markers 124 need not be in the forms exemplified in the present embodiment, and for example, at least some of the first stent markers 114 may be arranged in the similar forms to the second stent markers 124. Similarly, some of the second stent markers 124 may be arranged in the similar forms to the first stent markers 114.

### (Fifth Embodiment)

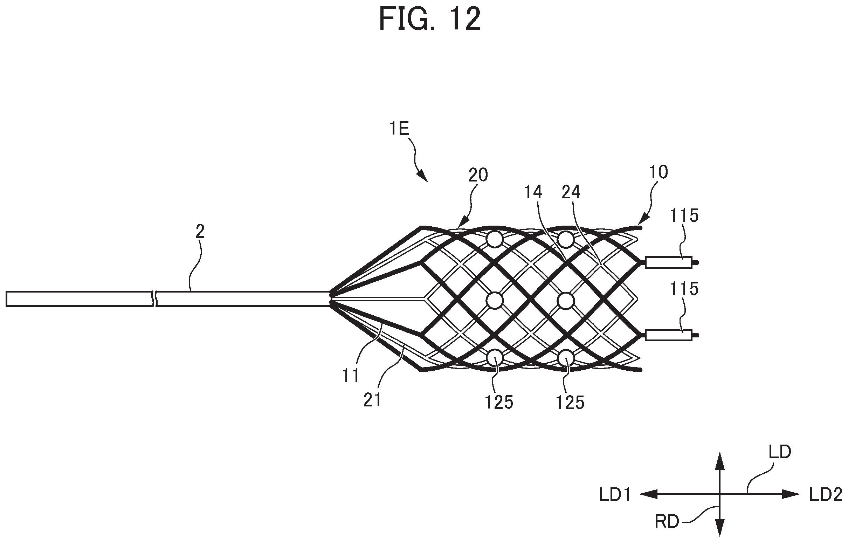

FIG. 12 is a schematic side view of a stent 1E of a fifth embodiment. The stent 1E of the fifth embodiment is similar to the stent of the fourth embodiment except that a form of a second stent marker 125 is different from the second stent marker 124 of the fourth embodiment. Therefore, the same reference numerals are used as elements having functions similar to those of the above-described fourth embodiment, and overlapping description thereof will be omitted as necessary. Note that FIG. 12 shows a more simplified schematic diagram than FIG. 1 of the first embodiment.

A first stent marker 115 of the fifth embodiment is configured similarly to the first stent marker 114 of the fourth embodiment, and therefore the detailed description thereof will be omitted.

The second stent marker 125 is a member serving as a mark for checking the position and the state of a second stent body 20 under observation by radiation, and is formed of a material which is highly opaque to radiation. The second stent marker 125 is arranged on an overlapping portion 24 of a second stent body 20. Second stent markers 125 are arranged on all overlapping portions 24 that are adjacent to each other in the circumferential direction OD. Moreover, the second stent markers 125 are not arranged on all of the overlapping portions 24 in the axial direction LD. In the present embodiment, the second stent markers 125 are arranged on the overlapping portions 24 of two rows in the vicinity of the central area. Note that a second stent marker 125 may be arranged on each of the overlapping portions 24.

In the fifth embodiment, the first stent marker 115 and the second stent marker 125 are arranged at different positions in the axial direction LD, and further, the forms (shape, size, and orientation) to be observed are also greatly different from each other. This makes it possible to visually distinguish and observe the first stent marker 115 and the second stent marker 125 more reliably under observation by radiation. Note that all of the first stent markers 115 and the second stent markers 125 need not be in the forms exemplified in the present embodiment, and for example, at least some of the first stent markers 115 may be arranged in the similar forms to the second stent markers 125. Similarly, some of the second stent markers 125 may be arranged in the similar forms to the first stent markers 115.

### (Sixth Embodiment)

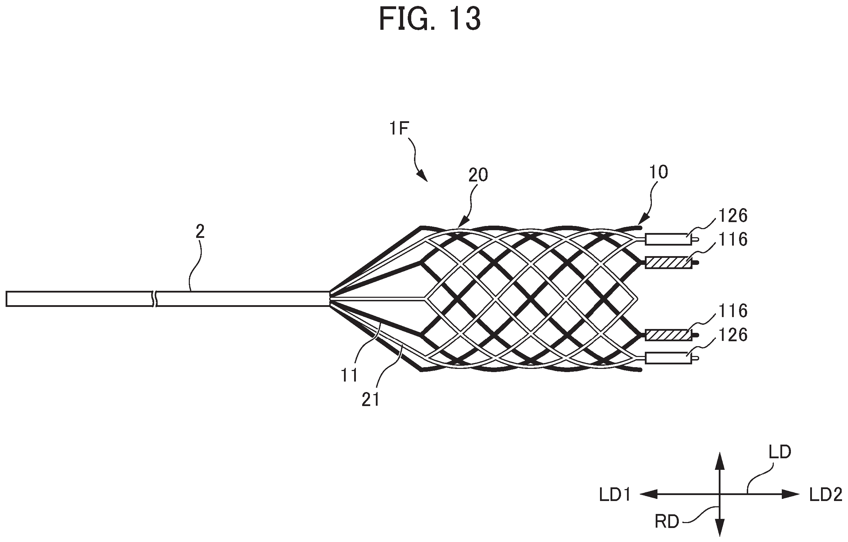

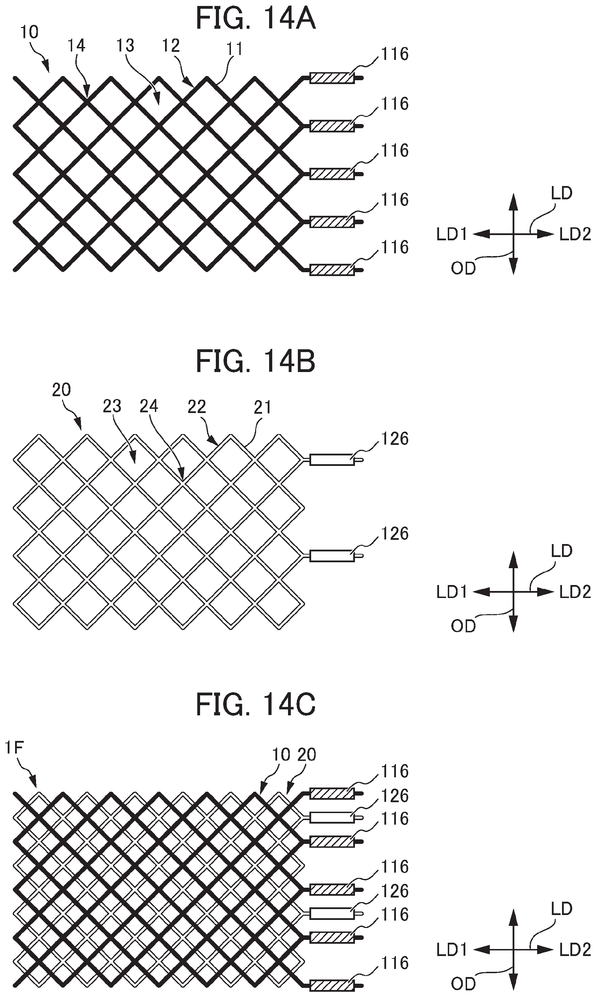

FIG. 13 is a schematic side view of a stent 1F of a sixth embodiment. FIG. 14A is a development view showing a state in which a part of a first stent body 10 of the sixth embodiment is virtually opened in a planar shape. FIG. 14B is a development view showing a state in which a part of a second stent body 20 of the sixth embodiment is virtually opened in a planar shape. FIG. 14C is a development view showing a state in which a part of the stent 1F of the sixth embodiment is virtually opened in a planar shape. The stent 1F of the sixth embodiment is similar to the stent of the fifth embodiment except that a form of a first stent marker 116 and a second stent marker 126 are different from the first stent marker 115 and the second stent marker 125 of the fifth embodiment. Therefore, the same reference numerals are used as elements having functions similar to those of the above-described fifth embodiment, and overlapping description thereof will be omitted as necessary. Note that FIGS. 13, and 14A to 14C show more simplified schematic diagrams than FIG. 1 and the like of the first embodiment.

The first stent marker 116 is a member serving as a mark for checking the position and the state of the first stent body 10 under observation by radiation, and is formed of a material which is highly opaque to radiation. The first stent marker 116 is a marker substantially similar to the first stent marker 111 of the first embodiment, and is a crimping marker that is crimp-fixed to the distal LD2 side end portion of the first stent body 10. First stent markers 116 are provided at the same interval as the interval (cycle) at which outer cells 12 are arranged in the circumferential direction OD.

The second stent marker 126 is a member serving as a mark for checking the position and the state of the second stent body 20 under observation by radiation, and is formed of a material which is highly opaque to radiation. The second stent marker 126 is a marker substantially similar to the second stent marker 121 of the first embodiment, and is a crimping marker that is crimp-fixed to the distal LD2 side end portion of the second stent body 20. Second stent markers 126 are provided side by side in the circumferential direction OD at different intervals and different numbers from the first stent markers 116. Specifically, the second stent markers 126 are provided at an interval twice as large as the interval at which inner cells 22 are arranged in the circumferential direction OD, that is, at an interval twice as large as the interval at which the first stent markers 116 are arranged in the circumferential direction OD. Therefore, the number of the second stent markers 126 is also different from the number of the first stent markers 116. In the present embodiment, the first stent markers 116 are provided at six places, and the second stent markers 126 are provided at three places.

In the sixth embodiment, the interval and the number of the first stent markers 116 arranged in the circumferential direction OD are different from the interval and the number of the second stent markers 126 arranged in the circumferential direction OD. Accordingly, it is possible to visually distinguish and observe the first stent markers 116 and the second stent markers 126 under observation by radiation.

### (Method For Capturing Image)

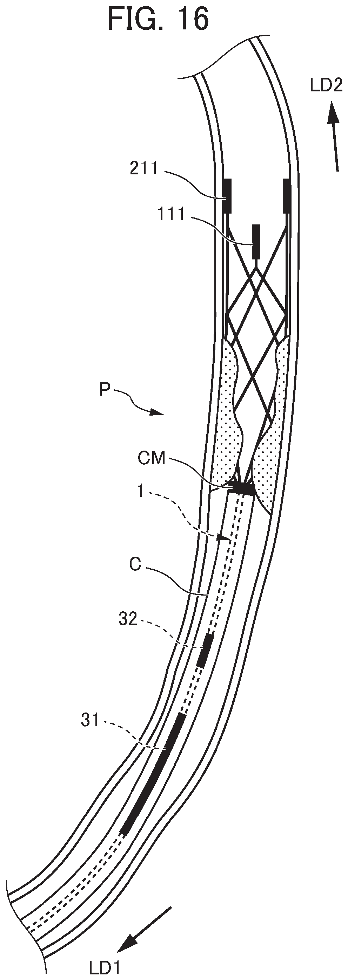

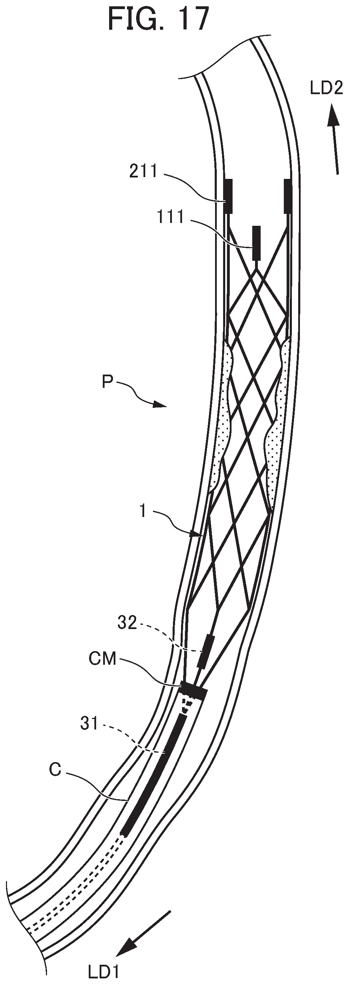

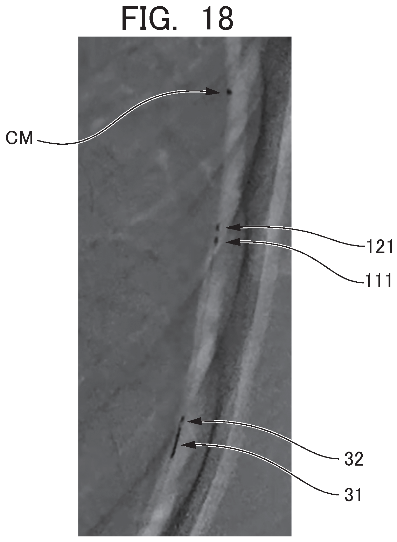

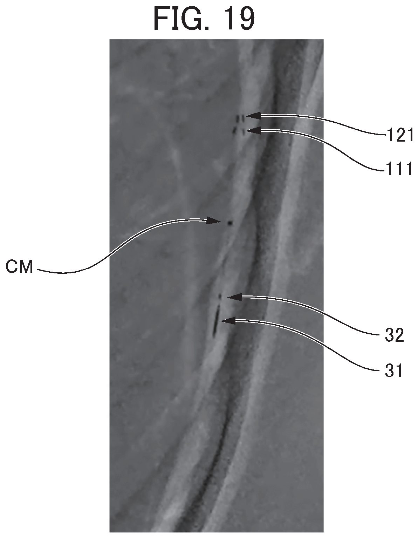

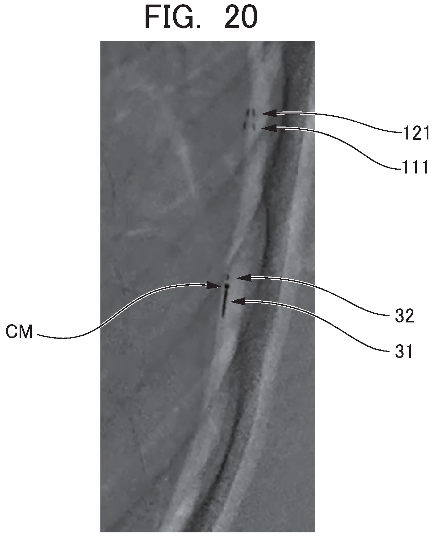

Next, to perform observation by radiation, a method for capturing an image of a stent of the present invention will be described. FIG. 15 is a diagram schematically showing an example of an image captured in capturing an image of a second stent marker 121 in a diameter-narrowed state of a stent 1 in a position where the stent 1 is to be expanded in the living body lumen. FIG. 16 is a diagram schematically showing an example of an image captured in capturing an image of the second stent marker 121 in an intermediate state while transitioning from a diameter-narrowed state to a diameter-expanded state of the stent 1 in the position where the stent 1 is to be expanded in the living body lumen. FIG. 17 is a diagram schematically showing an example of an image captured in capturing an image of the second stent marker 121 in a diameter-expanded state of the stent 1 in the position where the stent 1 is expanded in the living body lumen. FIG. 18 is a diagram showing an example of an image captured in capturing an image in a diameter-narrowed state of the stent. FIG. 19 is a diagram showing an example of an image captured in capturing an image while transitioning a state of the stent. FIG. 20 is a diagram showing an example of an image captured in capturing an image in a diameter-expanded state of the stent.

Here, a case will be described where a stent 1 of the first embodiment is used to expand the blood vessel, but an image can be captured similarly for the case where stents of other embodiments are used. Moreover, the distal LD2 side of a catheter C includes a catheter marker CM which is highly opaque to X-rays so that the position can be visually recognized under observation by X-rays. Note that in the examples shown in FIGS. 15 to 20, a shift amount of the second stent marker 121 arranged closer on the distal LD2 side than the first stent marker 111 is greater than that in FIG. 1 of the first embodiment.

When the blood vessel is expanded, the stent 1 sheathed in the catheter C in a state in which the stent is narrowed as shown in FIGS. 15 and 18 is delivered together with the catheter C through the blood vessel to a position P where the blood vessel is to be expanded. The position P where the blood vessel is to be expanded is, for example, a portion where an inner diameter of the blood vessel is smaller than other portions due to stenosis (narrowing). At this time, a practitioner captures an X-ray transmission video of the first stent marker 111 and the second stent marker 121 using an X-ray imaging apparatus (capturing image in diameter-narrowed state of stent). The first stent marker 111 and the second stent marker 121 are arranged on the distal LD2 side of the stent 1 of the first embodiment, so that the relative positional relationship between these markers and the catheter marker CM can be checked. Therefore, it is possible to check that the stent 1 is adequately sheathed in the catheter C and delivered to a desired position.

Next, when the catheter C is pulled toward the proximal LD1 side while preventing the push wire 2 from moving in the direction LD to maintain the position of the stent 1 in the blood vessel, the stent 1 starts to expand from the distal LD2 side (see FIGS. 16 and 19). At this time, the practitioner captures an X-ray transmission video of the first stent marker 111 and the second stent marker 121, and a first connection portion marker 31 and a second connection portion marker 32 using an X-ray imaging apparatus (capturing image while transitioning the stent). It is possible to check the positions of the first stent marker part 111 and the second stent marker part 121 relative to the catheter marker CM, and the positions of the first connection portion marker 31 and the second connection portion marker 32 relative to the catheter marker CM on the basis of the imaging results. Moreover, the second stent marker 121 is arranged closer on the distal LD2 side than the first stent marker 111, so that it is possible to distinguish and recognize the first stent marker 111 and the second stent marker 121. Therefore, it is possible to visually check that the expansion of the stent 1 adequately advances, the position of the stent 1 is not moved, and the expansion of the stent 1 is started.

When the expansion of the stent 1 is completed, the state shown in FIGS. 17 and 20 is achieved. At this time, a practitioner captures X-ray transmission videos of the first stent marker 111, the second stent marker 121, the first connection portion marker 31 and the second connection portion marker 32 using an X-ray imaging apparatus (capturing image in diameter-expanded state of stent). Similarly to the step of the capturing an image during the transition of a stent, , , it is possible to distinguish and recognize the first stent marker 111 and the second stent marker 121 in the step of capturing an image in the diameter-expanded state of the stent since the second stent marker 121 is arranged closer on the distal LD2 side than the first stent marker 111. Moreover, the first connection portion marker 31 and the second connection portion marker 32 are arranged at a predetermined interval. Accordingly, it is possible to check that the catheter C is reliably pulled from the range in which the stent 1 expands, when the catheter marker CM is located at a position for an interval portion between the first connection portion marker 31 and the second connection portion marker 32. Therefore, it can be visually checked that the expansion of the stent 1 has been adequately completed at an appropriate position. Note that when the expanded stent 1 is resheathed in the catheter C, the above-described operation and capturing image are executed in reverse order. Note that also when the stent 1 is resheathed in the catheter C, an X-ray transmission video of the first stent marker 111 and the second stent marker 121 is captured using the X-ray imaging apparatus (capturing image in diameter-narrowed state of stent).

### (Modification)

The present disclosure is not limited to the above-described embodiments and various modifications and changes can be made, and these modifications and changes are also included in the scope of the present disclosure.
(1) In each embodiment, a specific form of a marker has been described as an example. However, the specific form of the marker in each embodiment is not limited to the example shown in the embodiment, and conventionally known markers in various forms may be available. For example, a portion of the crimping marker may be replaced with a coil marker or radiopaque strut.
(2) In each embodiment, the description has been made in which the marker exists also on the proximal LD1 side. The present invention is not limited thereto, and for example, the marker on the proximal LD1 side may be omitted.
(3) Each embodiment may be combined to further clarify a difference between the first stent marker and the second stent marker . For example, in the first embodiment, at least one of the shape, size, and orientation of the first stent marker 111 may be different from that of the second stent marker 121.
(4) In each embodiment, a configuration having both of the first stent marker and the second stent marker has been described as an example. The present invention is not limited thereto, and for example, the first stent marker may be omitted. In the stent having a double-layer structure, when the second stent body 20 is adequately expanded, the first stent body 10 is also expanded adequately. Therefore, even when only the second stent marker is observed, it can be determined that both of the first stent body and the second stent body are adequately expanded. Therefore, in the stent having a double-layer stent, at least the second stent body may be provided with the second stent marker.
(5) In the fourth and fifth embodiments, an example has been described in which the shape of the first stent marker (114, 115) is different from that of the second stent marker (124, 125). The present invention is not limited thereto, and the size and orientation of the first stent marker are configured to be different from those of the second stent marker , and therefore, both stent markers can be distinguished from each other and visually recognized. In this case, the shape of the first stent marker may be the same as that of the second stent marker . That is, when the first stent marker differs from the second stent marker in at least one of the shape, size, and orientation, both stent markers can be distinguished from each other and visually recognized.

Note that each embodiment and modification may be combined as necessary, but detailed description thereof will be omitted. The present disclosure is not limited to each embodiment described above.

### EXPLANATION OF REFERENCE NUMERALS

1, 1B, 1C, 1D, 1E, 1F Stent
2 Push wire
3 Distal end shaft
10 First stent body
11 Strut
11a Slit
12 Outer cell
13 Opening portion
14 Overlapping portion
15 Curved portion
20 Second stent body
20A Second stent body
21 Strut
22 Inner cell
23 Opening portion
24 Overlapping portion
25 Curved portion
31 First connection portion marker
32 Second connection portion marker
111, 112, 113, 114, 115, 116 First stent marker
121, 122, 123, 124, 125, 126 Second stent marker
C Catheter
CM Catheter marker

## Claims

1. A stent for expanding a living body lumen, comprising:
a first stent body in which a plurality of first cells surrounded by a plurality of first struts are arrayed in a circumferential direction and a central axis direction;
a second stent body in which a plurality of second cells surrounded by a plurality of second struts are arrayed in the circumferential direction and the central axis direction and at least a region for expanding a living body lumen is inserted into the first stent body, wherein
at least a part of the second stent body includes a second stent marker which is highly opaque to radiation.

2. The stent according to claim 1, wherein
at least a part of the first stent body includes a first stent marker which is highly opaque to radiation, and
at least a part of the first stent marker is different positionally from the second stent marker in an axial direction of the stent.

3. The stent according to claim 1, wherein
at least a part of the first stent body includes a first stent marker which is highly opaque to radiation, and
at least a part of the first stent marker is different from the second stent marker in at least one of an interval and a number with which each of the first and second stent markers is arranged in the circumferential direction of the stent.

4. The stent according to claim 2 or 3, wherein
at least a part of the first stent marker is different from the second stent marker in at least one of shape, size, and orientation.

5. A method for capturing an image of the stent according to claim 1 in a living body lumen, the method comprising:
capturing an image of the second stent marker in a diameter-narrowed state of the stent at a position where the stent is to be expanded in the living body lumen; and
capturing an image of the second stent marker in a diameter-expanded state of the stent.
